Europäisches Patentamt

European Patent Office

Office européen des brevets

(19)

(11)  **EP 1 139 978 B1**

(12)  **EUROPÄISCHE PATENTSCHRIFT**

(45) Veröffentlichungstag und Bekanntmachung des Hinweises auf die Patenterteilung:
**26.10.2005   Patentblatt 2005/43**

(51) Int Cl.⁷: **A61K 7/00**, A61K 7/42

(21) Anmeldenummer: **99958139.0**

(86) Internationale Anmeldenummer:
**PCT/EP1999/009291**

(22) Anmeldetag: **30.11.1999**

(87) Internationale Veröffentlichungsnummer:
**WO 2000/038622 (06.07.2000 Gazette 2000/27)**

(54) **SONNENSCHUTZMITTEL MIT MIKROPARTIKELN AUF BASIS VON WASSERUNLÖSLICHEM LINEAREM POLYGLUCAN**

SUN PROTECTION PRODUCT WITH MICROPARTICLES ON THE BASIS OF WATER-INSOLUBLE LINEAR POLYGLUCAN

PRODUIT SOLAIRE COMPRENANT DES MICROPARTICULES A BASE DE POLYGLUCANE LINEAIRE SOLUBLE DANS L'EAU

(84) Benannte Vertragsstaaten:
**AT BE CH CY DE DK ES FI FR GB GR IE IT LI LU MC NL PT SE**

(30) Priorität: **28.12.1998   DE 19860368**

(43) Veröffentlichungstag der Anmeldung:
**10.10.2001   Patentblatt 2001/41**

(73) Patentinhaber: **SÜDZUCKER AKTIENGESELLSCHAFT**
**68165 Mannheim (DE)**

(72) Erfinder:
• **BENGS, Holger**
  **D-60598 Frankfurt am Main (DE)**
• **BRAUNAGEL, Alfred**
  **D-55128 Mainz (DE)**

(74) Vertreter: **Luderschmidt, Schüler & Partner GbR Patentanwälte**
**Industriepark Höchst**
**65926 Frankfurt (DE)**

(56) Entgegenhaltungen:
**WO-A-94/18932        WO-A-97/28780**
**WO-A-99/11695        DE-C- 19 839 216**
**GB-A- 2 247 242      US-A- 5 486 507**

• INABA, R. ET AL.: "Application of porous starch complex powder" STN INTERNATIONAL, FILE CAPLUS, AN1995:883105, XP002130221
• PATENT ABSTRACTS OF JAPAN vol. 1999, no. 14, 22. Dezember 1999 (1999-12-22) & JP 11 246379 A (KOSE CORP), 14. September 1999 (1999-09-14)

Anmerkung: Innerhalb von neun Monaten nach der Bekanntmachung des Hinweises auf die Erteilung des europäischen Patents  kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Sonnenschutzmittel mit Mikropartikeln auf Basis von wasserunlöslichen linearen Polyglucan, das einerseits einen guten UV-Schutz gewährleistet und bei der Applikation transparent erscheint.

Bekannte Sonnenschutzmittel enthalten unter anderem Pigmente wie z.B. Titandioxid und Zinkoxid als solche oder in Form von sogenannten mikronisierten Partikeln als Lichtschutzfilter.

Diese Pigmente haben vom kosmetischen Gesichtsichtspunkt aus den Nachteil, daß sie weißeln, d.h. die Haut weiß gefärbt erscheinen lassen.

Als Kompromiß zur Verringerung des Weißelns auf ein erträgliches Maß und dennoch Erzielen eines akzeptablen UV-Schutzes werden diese Pigmente üblicherweise mit vergleichsweise kleiner Teilchengröße zwischen 10 und 100 nm eingesetzt (A. Schrader, M. Rohr "Auffälligkeiten bei der Entwicklung und Prüfung von mikropigmenthaltigen Sonnenschutzformulierungen" SÖFM-Journal, 124, Seiten 478-487, 8/98).

Titandioxid ist zudem unter dem gesundheitlichen Aspekt kritisch zu betrachten. In einer Studie mit Titandioxid, das aus Sonnenschutzmittel extrahiert worden ist, wurde bei Bestrahlung mit Sonnenlicht beobachtet, daß Titandioxid Photooxidation zu katalysieren vermag und Bakterien-DNA schädigt (CTFA/TRN Volume 12, No. 3, Seite 5 (1998) mit Verweis auf FEBS Letters, 418, 87-90, 1997).

Eine lichtschützende Wirkung ist auch für einzelne Polysaccharide bekannt. So ist für Poly-$\beta$-1,3-Glucane eine lichtschützende Wirkung beschrieben (H. Eggensperger, M. Wilker, "Multiaktiv wirksame Polysaccharide Teil I-Pilzextrakte und Teil II-Pflanzliche Polysaccharide"in SÖFW-Journal, 123, 8/97, Seiten 542-546 und 12/97, Seiten 838-842).

Poly-$\beta$-1,3-Glucane, die aus Hefen erhalten werden können, besitzen eine lineare Struktur mit einem geringen Anteil an $\beta$-1,6-Verzweigung.

Weiter wird vorgeschlagen, biotechnisch oder aus marinen Mollusken gewonnenes Glykogen, ein hochverzweigtes Poly-1,4-$\alpha$-glucan mit Verzweigungen in 6-Position, für Sonnenschutzmittel einzusetzen (M. Pauly, G. Pauly "New Polysaccharides Interest in Care Cosmetology" IN-COSMETICS 1997, Conference Proceedings, Seiten 417-444, Verlag für chemische Industrie, H.Ziolkowsky GmbH, 1998.

In der EP-B-0 487 000 wird die Verwendung einer emulsionsförmigen kosmetischen Zusammensetzung mit 15 bis 40 Gew.% einer enzymatisch entzweigten Stärke in Sonnenschutzmittel vorgeschlagen, wobei die enzymatisch abgebaute Stärke ein lineares Poly-1,4-$\alpha$-glucan mit 15 bis 65 Anhydroglucoseeinheiten ist. Es findet sich jedoch kein Hinweis auf eine mögliche Lichtschutzwirkung der dort verwendeten enzymatisch entzweigten Stärke, vielmehr wird sie als Emulgierhilfsmittel eingesetzt.

In Hinblick auf die Risiken einer intensiven UV-Belastung besteht ein zunehmender Bedarf an geeigneten Sonnenschutzfiltern, die nicht nur einen zuverlässigen Schutz gewähren sondern zudem das äußere Erscheinungsbild nicht beeinträchtigen und damit auch für die tägliche Anwendung geeignet sind.

Erfindungsgemäß wird diese Aufgabe durch die Verwendung gemäß Anspruch 1 gelöst.

Die erfindungsgemäß als wirksamer Bestandteil in Sonnenschutzmitteln verwendeten sphärischen Mikropartikel, die ganz oder teilweise aus mindestens einem wasserunlöslichen linearen Polyglucan bestehen, können einen ausgezeichneten UV-Schutz gewährleisten und zudem erscheinen die Mikropartikel selbst in hohen Konzentrationen transparent.

Daneben bilden die erfindungsgemäß verwendeten Mikropartikel selbst ohne Zusatz von Dispergierhilfsmitteln stabile Suspensionen oder Dispersionen aus. Dies ist insbesondere für die Anwendung in Sonnenschutzmitteln auf Emulsionsbasis von R. INABA, et al. "Application of porous starch complex powder" STIN INTERNATIONAL, FILE CAPLUS, AN 1995: 883105 offenbart ein Sonnenschutzmitteln, welches Stärkepartikel mit einem miltleren Durchmesser von 10µm enthält.

Vorteil, da auf den Zusatz von Dispergierhilfsmitteln verzichtet oder deren Menge verringert werden kann, und somit die Produktion vereinfacht und verbilligt wird.

Zudem vermitteln die sphärischen Mikropartikel beim Auftragen ein angenehmes weiches Gefühl, was auf ihre regelmäßige Gestalt zurückgeführt wird.

Für die Sonnenschutzmittel kann auf die für derartige Mitteln üblichen Formulierungen und Zusätze zurückgegriffen werden. Besonders bevorzugte Grundlagen für Formulierungen sind Emulsionen wie z.B. o/w- oder w/o-Emulsionen, wässerige oder fetthaltige Gele, Hydrogele, Öle, emulgatorfreie Formulierungen etc.. Die Sonnenschutzmittel können in Form von Cremes, Lotionen, Sprays, Fluiden, Puder etc. angewendet werden.

Die Sonnenschutzmittel können neben den Mikropartikeln noch weitere, bekannte UV-Filter enthalten.

Der Anteil der Mikropartikel in den Sonnenschutzmitteln richtet sich nach der verwendeten Grundlage. Er kann bis zu 70 Gew.%, bezogen auf das Gesamtgewicht des Sonnenschutzmittels betragen z.B. in Wachs-Öl-Grundlagen wie sie unter anderem für sogenannte "Sun Protection Cream Compacts" verwendet werden.

Im allgemeinen genügen Mengen von etwa 0,5 bis etwa 20 Gew.%, vorzugsweise etwa 2 bis etwa 15 Gew.% und insbesondere etwa 3 bis etwa 10 Gew.%.

Eine geringere Menge als 0,5 Gew.% ist für einen UV-Schutz ohne Bedeutung.

Es versteht sich, daß die Menge stark von der Zusammensetzung des Mittels abhängt.

Enthält das Sonnenschutzmittel weitere UV-Filter oder ist die Grundlage des Sonnenschutzmittels an sich gefärbt oder pigmentiert wie z.B. pigmentierte w/o- oder o/w-Emulsionen, so daß eine verringerte UV-Durchlässigkeit von vorneherein gegeben ist, können kleinere Mengen an Mikropartikeln ausreichend sein.

Für UV-durchlässige Grundlagen, z.B. transparente Grundlagen wie z.B. unpigmentierte Emulsionen, Öle oder Gele, werden zweckmäßigerweise größere Mengen an Mikropartikeln zugesetzt.

Der benötigte Menge kann von einem Fachmann mit wenigen Routineversuchen jedoch ohne weiteres von Fall zu Fall bestimmt werden.

Die für die erfindungsgemäßen Sonnenschutzmittel eingesetzten Mikropartikel können einen mittleren Durchmesser Dn (Zahlenmittelwert) von 1 nm -100 μm vorzugsweise 50 nm - 10 μm, insbesondere 100 nm - 3 μm und besonders bevorzugt kleiner 1 μm, insbesondere kleiner 0,2 μm, haben

Für die vorliegende Erfindung sind unter sphärischen Mikropartikeln Mikropartikel zu verstehen, die annähernd Kugelform besitzen. Bei Beschreibung einer Kugel durch von einem gemeinsamen Ursprung ausgehende, in den Raum gerichtete Achsen gleicher Länge, die den Radius der Kugel in allen Raumrichtungen definieren, ist für die sphärischen Partikel eine Abweichung der Achsenlängen vom Idealzustand der Kugel von 1 % bis 40 % möglich. Bevorzugt beträgt die Abweichung 25 % oder weniger, besonders bevorzugt 15 % oder weniger.

Die sphärischen Partikel haben eine regelmäßige Oberfläche, die makroskopisch mit einer Himbeere verglichen werden kann, wobei die Tiefe von Unregelmäßigkeiten auf der Partikeloberfläche, wie Eindellungen oder Einschnitte, maximal 20 %, vorzugsweise 10 %, des mittleren Durchmessers der sphärischen Mikropartikel beträgt.

Die spezifische Oberfläche der Mikropartikel beträgt im allgemeinen von 1 m²/g bis 100 m²/g, vorzugsweise 1,5 m²/g bis 20 m²/g und besonders bevorzugt 3 m²/g bis 10 m²/g.

Weiter zeigen die erfindungsgmäßen Partikel eine Dispersität $D$ = Gewichtsmittelwert des Durchmessers $(d_w)$ / Zahlenmittelwert des Durchmessers $(d_n)$ von 1,0 bis 10,0, insbesondere von 1,5 bis 5,0 und besonders bevorzugt von 2,0 bis 3,0.

Die hier benutzten Mittelwerte sind wie folgt definiert:

$$d_n = \text{Summe } n_i \times d_i / \text{Summe } n_i = \text{Zahlenmittelwert}$$

$$d_w = \text{Summe } n_i \times d_i^2 / \text{Summe } n_i \times d_i = \text{Gewichtsmittelwert}$$

$$n_i = \text{Anzahl der Partikel mit Durchmesser } d_i,$$

$$d_i = \text{ein bestimmter Durchmesser,}$$

$$i = \text{fortlaufender Parameter.}$$

In diesem Zusammenhang bedeutet der Begriff Gewicht ein gewichtetes Mittel, wodurch die größeren Durchmesser einen höheren Stellenwert erhalten.

Die erfindungsgemäß verwendeten Mikropartikel können auch einer Oberflächenmodifikation unterzogen worden sind, indem z.B. funktionelle Gruppen wie Hydroxylgruppen der Polyglucane derivatisiert werden.

Lineare wasserunlösliche Polyglucane im Sinne der vorliegenden Erfindung sind Polysaccharide, die aus Glucanen als monomeren Bausteinen derart aufgebaut sind, daß die einzelnen Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind lediglich die beiden Grundeinheiten, die den Anfang bzw. das Ende des Polysaccharids bilden. Diese haben nur eine Verknüpfung zu einem weiteren Monomer und bilden die Endgruppen des linearen Polyglucans.

Besitzt die Grundeinheit drei oder mehr Verknüpfungen, wird von Verzweigung gesprochen. Dabei ergibt sich aus der Anzahl der Hydroxylgruppen pro 100 Grundeinheiten, die nicht am Aufbau des linearen Polymerrückgrats beteiligt sind und die Verzweigungen ausbilden, der sogenannte Verzweigungsgrad. Erfindungsgemäß weisen die linearen wasserunlöslichen Polyglucane einen Verzweigungsgrad von maximal 8 % auf, d.h. sie haben maximal 8 Verzweigungen auf 100 Grundeinheiten. Vorzugsweise ist der Verzweigungsgrad kleiner 4 % und insbesondere maximal 2,5 %.

Besonders bevorzugt sind Polyglucane deren Verzweigungsgrad in 6-Position kleiner 4 %, vorzugsweise maximal 2 % und insbesondere maximal 0,5 %, und in den anderen Positionen, z. B. in 2- bzw. 3-Position, vorzugsweise jeweils maximal 2 % und insbesondere 1 % ist. Besonders bevorzugt sind auch Polyglucane mit einem Verzweigungsgrad in 6-Position kleiner 0,5 %.

Für die Erfindung sind insbesondere Polyglucane geeignet, die keine Verzweigungen aufweisen, bzw. deren Verzweigungsgrad so minimal ist, daß er mit herkömmlichen Methoden nicht mehr nachweisbar ist

Beispiele für bevorzugte wasserunlösliche lineare Polyglucane sind lineare Poly-D-glucane, wobei die Art der Verknüpfung unwesentlich ist, solange Linearität im Sinne der Erfindung vorliegt. Beispiele sind Poly-alpha-D-glucane, insbesondere Poly-1,4-alpha-D-glucan, und Poly-1,3-beta-D-glucane, wobei Poly-1,4-α-D-glucan besonders bevorzugt ist.

Für die vorliegende Erfindung beziehen sich die Präfixe "alpha", "beta" oder "D" allein auf die Verknüpfungen, die das Polymerrückgrat ausbilden und nicht auf die Verzweigungen.

Unter dem Begriff "wasserunlösliches Polyglucan" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag, Frankfurt, Auflage, 1987) entsprechend den Klassen 4 bis 7 unter die Kategorien "wenig lösliche", "schwer lösliche", "sehr schwer lösliche" bzw. "praktisch unlösliche" Verbindungen fallen.

Im Fall der erfindungsgemäß verwendeten Polyglucane bedeutet dies, daß mindestens 98 % der eingesetzten Menge, insbesondere mindestens 99,5 %, unter Normalbedingungen (T = 25 °C +/- 20 %, p= 101325 Pascal +/- 20 %) in Wasser unlöslich ist (entsprechend den Klassen 4 bzw. 5).

Für die vorliegende Erfindung sind schwer lösliche bis praktisch unlösliche Verbindungen, insbesondere sehr schwer lösliche bis praktisch unlösliche Verbindungen, bevorzugt.

"Sehr schwer löslich" entsprechend Klasse 6 kann durch folgende Versuchsbeschreibung veranschaulicht werden:

Ein Gramm des zu untersuchenden Polyglucans werden in 1 l entionisierten Wasser auf 130° C unter einem Druck von 1 bar erhitzt. Die entstehende Lösung bleibt nur kurzzeitig über wenige Minuten stabil. Beim Erkalten unter Normalbedingungen fällt die Substanz wieder aus. Nach Abkühlung auf Raumtemperatur und Abtrennung mittels Zentrifugation können unter Berücksichtigung der experimentellen Verluste mindestens 66 % der eingesetzten Menge zurückgewonnen werden.

Die erfindungsgemäß eingesetzten Polyglucane können beliebigen Ursprungs sein, solange die vorstehend angegebenen Bedingungen in bezug auf die Begriffe "linear" und "wasserunlöslich" erfüllt sind.

Sie können natürlich oder auf biotechnischen Wege gewonnen sein.

Beispielsweise können sie aus natürlichen pflanzlichen oder tierischen Quellen durch Isolierung und/oder Aufreinigung erhalten werden. Es können auch Quellen zum Einsatz kommen, die gentechnisch derart manipuliert worden sind, daß sie im Vergleich zu der unmanipulierten Quelle einen höheren Anteil an nicht oder vergleichsweise geringfügig verzweigten Polyglucanen enthalten.

Sie können durch enzymatische oder chemische Entzweigung aus nicht-linearen Polyglucanen hergestellt worden sein. Dabei können nicht-lineare Polyglucane, die Verzweigungen enthalten, derart mit einem Enzym behandelt werden, daß es zur Spaltung der Verzweigungen kommt, so daß nach ihrer Abtrennung lineare Polyglucane vorliegen. Bei diesen Enzymen kann es sich beispielsweise um Amylasen, iso-Amylasen, Gluconohydrolasen, Pullulanasen oder Cyclomaltodextrin-glucanotransferasen handeln.

Biotechnische Methoden umfassen biokatalytische, auch biotransformatorische, oder fermentative Prozesse.

Lineare Polyglucane hergestellt durch Biokatalyse (auch: Biotransformation) im Rahmen dieser Erfindung bedeutet, daß das lineare Polyglucan durch kataly-tische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird. Man spricht in diesem Zusammenhang auch von "in vitro Biokatalyse".

Linerare Polyglucane aus Fermentationen sind im Sprachgebrauch der Erfindung lineare Polyglucane, die durch fermentative Prozesse unter der Verwendung in der Natur vorkommende Organismen, wie Pilzen, Algen, Bazillen, Bakterien oder Protisten oder unter der Verwendung von in der Natur nicht vorkommender Organismen, aber unter Zuhilfenahme von gentechnischen Methoden allgemeiner Definition modifizierten natürlichen Organismen, wie Pilzen, Algen, Bazillen, Bakterien oder Protisten gewonnen werden oder unter Einschaltung und Mithilfe von fermentativen Prozessen gewonnen werden können. Man spricht in diesem Zusammenhang auch von "in vivo Biokatalyse".

Beispiele für derartige Mikroorganismen sind Piichia pastoris, Trichoderma Reseii, Straphylokkus Carnosus, Escherichia Coli, Aspergillus Niger.

Vorteilhafte Verfahren für die biotechnische Gewinnung sind z. B. in der WO 95/31553 oder der nicht vorveröffentlichten deutschen Patentanmeldung der Anmelderin mit amtlichen Aktenzeichen 198 27 978.5 beschrieben.

Gemäß der Wo 95/31553 werden Amylosucrasen zur Herstellung von linearen wasserunlöslichen Polyglucan wie Poly-1,4-$\alpha$-D-glucan mittels eines biokatalytischen Verfahrens verwendet. Weitere geeignete Enzyme sind Polysaccharidsynthasen, Stärkesynthasen, Glycoltransferasen, 1,4-$\alpha$-D-Glucantransferasen, Glycogensynthasen oder auch Phosphorylasen.

Es können auch modifizierte wasserunlösliche lineare Polyglucane eingesetzt werden, wobei die Polyglucane beispielsweise durch Veresterung und/oder Veretherung in einer oder mehreren nicht an der linearen Verknüpfung beteiligten Positionen chemisch modifiziert worden sein können. Im Fall der bevorzugten 1,4 verknüpften Polyglucane kann die Modifizierung in 2-, 3- und/oder 6-Position erfolgen.

Modifikation im Sinne der Erfindung bedeutet, daß die vorhandenen Hydroxylgruppen, die nicht an der Verknüpfung beteiligt sind, chemisch verändert werden. Dies schließt eine Ringöffnung der Glucaneinheiten aus wie sie z.B. bei der oxidativen Carboxylierung oder der Hydrolyse erfolgt. Maßnahmen für derartige Modifizierungen sind dem Fachmann hinlänglich bekannt.

So können lineare Polyglucane wie z.B. Pullulane, die an sich wasserlöslich sind, durch Modifizierung wasserunlöslich gemacht werden.

Für die vorliegende Erfindung werden bevorzugt wasserunlösliche lineare Polyglucane eingesetzt, die in einem biotechnischen, insbesondere in einem biokatalytischen oder einem fermentativen Prozeß hergestellt worden sind.

Im Gegensatz zu Polyglucanen, die aus natürlichen Quellen, wie Pflanzen, isoliert werden, weisen die hierbei erhaltenen linearen wasserunlöslichen Polyglucane ein besonders homogenes Eigenschaftsprofil auf, z. B. in bezug auf die Molekulargewichtsverteilung, sie enthalten keine oder allenfalls nur in sehr geringen Mengen unerwünschte Nebenprodukte, die aufwendig abgetrennt werden müssen oder allergene Reaktionen auslösen könnten, und lassen sich exakt spezifiziert auf einfache Weise reproduzieren.

Insbesondere können mit biotechnischen Methoden wasserunlösliche lineare Polyglucane erhalten werden, wie z. B. die bevorzugten Poly-1,4-$\alpha$-D-gluca-ne, die keine Verzweigungen enthalten, bzw. deren Ver-zweigungsgrad unterhalb der Nachweisgrenze her-kömmlicher analytischer Methoden liegt.

Weiter können die Polyglucane in Form soge-nannter alpha-amylaseresistenter Polyglucane einge-setzt werden wie sie am Beispiel von Poly-1,4-$\alpha$-D-glu-can in der prioritätsälteren, nicht vorveröffentlichten deutschen Patentanmeldung mit amtlichen Aktenzeichen 198 30 618.0 der Anmelderin beschrieben sind.

Alpha-amylaseresistente Polyglucane können durch Herstellung einer Suspension oder Dispersion aus wasserunlöslichen Polyglucanen und Wasser, Erwärmen der Suspension oder Dispersion auf eine Temperatur im Bereich von 50 bis 100 °C, Abkühlenlassen der erhaltenen kleisterartigen Mischung auf eine Temperatur im Bereich von 50 °C bis an den Gefrierpunkt, vorzugsweise 35 bis 15 °C, 27 bis 22 °C, 16 bis 0 °C oder 6 bis 2°C, über einen Zeitraum von 1 bis 72 h, vorzugsweise 1 bis 36 h und insbesondere 15 bis 30 h und Retrogradation der kleisterartigen Mischung bei einer gegenüber der Temperatur der erwärmten kleisterartigen Mischung erniedrigten Temperatur in einem Temperaturbereich von 90 bis 4 °C sowie gegebenenfalls Trocknung oder Entwässerung des erhaltenen Produktes erhaltenen werden.

Das Polyglucan kann auch als thermoplastisches Polyglucan eingesetzt werden, das erhältlich ist durch Aufschmelzen von linearem wasserunlöslichen Polyglucan und Hinzufügen von mindestens 20 Gew.%, vorzugsweise mindestens 30 Gew.%, eines Weichmachers wie Sorbitol, Glycerin, deren Kondensationsprodukte und Oligomere, DMSO, Bernsteinsäure, Citronensäure-Monohydrat, Apfelsäure, Weinsäure etc. bei ca. 170 °C.

Eine Beschreibung von geeigneten Maßnahmen und Eigenschaften von thermoplastischen Polyglucanen am Beispiel des bevorzugten linearen wasserun-löslichen Poly-1,4-$\alpha$-D-glucans gibt die prioritätsältere nicht vorveröffentlichte deutsche Patentanmeldung mit amtlichen Aktenzeichen 198 52 826, auf die hierfür ausdrücklich bezug genommen wird.

Die Molekulargewichte $M_w$ (Gewichtsmittel, bestimmt mittels Gelpermeationschromatographie im Vergleich zu einer Eichung mit Pullulanstandard) der erfindungsgemäß verwendeten linearen Polyglucane können in einem weiten Bereich von $0,75 \times 10^2$ g/mol bis $10^7$ g/mol variieren. Bevorzugt liegt das Molekulargewicht $M_w$ in einem Bereich von $10^3$ g/mol bis $10^6$ g/mol und besonders bevorzugt von $10^3$ g/mol bis $10^5$ g/mol. Ein weiterer vorteilhafter Bereich ist von $2 \times 10^3$ bis $8 \times 10^3$. Entsprechende Bereiche gelten für das bevorzugt eingesetzte Poly-1,4-D-glucan.

Die Molekulargewichtsverteilung bzw. Polydi-spersität $M_w/M_n$ kann ebenfalls in weiten Bereichen je nach Herstellungsverfahren des Polyglucans variieren. Bevorzugte Werte sind von 1,01 bis 50, insbesondere von 1,01 bis 15. Besonders bevorzugt sind Polyglucane mit kleinen Dispersitätswerten wie z.B. 1,01 - 2,5. Dabei nimmt die Polydispersität mit einer bimodalen Verteilung der Molekulargewichte zu.

Für die Herstellung der Mikropartikel kann ein einziges Polyglucan, insbesondere Poly-1,4-D-glucan und ganz besonders Poly-1,4-$\alpha$-D-glucan oder Mi-schungen aus zwei oder mehreren Vertretern verwendet werden.

In einer weiteren Ausführungsform kann ein wasserunlösliches verzweigtes Polysaccharid, vorzugsweise ein Polyglucan, insbesondere ein Poly-1,4-alpha-D-glucan oder ein Poly-1,3-beta-D-glucan, zugesetzt werden. Es können auch Gemische aus zwei oder mehreren verzweigten Polysacchariden zugegeben werden.

Die verzweigten Polysaccharide können beliebigen Ursprungs sein. In diesem Zusammenhang wird auf die diesbezüglichen Erläuterungen für die linearen wasserunlöslichen Polyglucane verwiesen. Bevorzugte Quellen sind Stärke und Stärkeanaloga wie Glykogen. Falls erforderlich kann in den verzweigten Polysacchariden der Anteil an linearen Strukturen durch geeignete Anreicherungsverfahren erhöht werden.

Für die Wasserunlöslichkeit gelten die gleichen Angaben wie für das lineare wasserunlösliche Polyglucan, das Molekulargewicht kann für die verzweigten Polysaccharide auch höher liegen, z. B. Werte bis vorzugsweise $10^9$ g/mol und mehr aufweisen.

Es können auch andere Polymere, insbesondere biokompatible oder bioabbaubare Polymere, beigemischt werden. Dabei hängt die Menge des oder der anderen Polymeren, die beigemengt werden, ohne daß die sphärische Gestalt und/oder sonstige Eigenschaften der herzustellenden Mikropartikel verändert werden, stets von dem zugesetzten Polymer ab.

Zur Sicherstellung der gewünschten Eigenschaften der Mikropartikel sollte der Anteil an linearen wasserunlöslichen Polyglucan mindestens 70 Gew.%, insbesondere 80 Gew.% und vorzugsweise 90 Gew.%, bezogen auf den Gesamtgehalt an linearem wasserunlöslichen Polyglucan inkl. ggf. verzweigtes Polysaccharid und gegebenenfalls weitere Polymere, betragen.

Gemäß einer besonders bevorzugten Ausführungsform bestehen die Mikropartikel zu 100 Gew.% aus linearem wasserunlöslichen Polyglucan, insbesondere linearen wasserunlöslichen Poly-1,4-α-D-glucan, das vorzugsweise biokatalytisch erhalten worden ist.

Beispiele für Verfahren zur Herstellung der Mikropartikel sind z. B. Fällprozeße oder Sprühtrocknungsverfahren.

Die Herstellung der sphärischen Mikropartikel kann durch Lösen des wasserunlöslichen linearen Polyglucans oder einer Mischung von mehreren davon sowie gegebenenfalls weiterer Polymere in einem Lösungsmittel , z. B. DMSO , Einbringen der Lösung in ein Fällmittel , z. B. Wasser, vorzugsweise bei einer Temperatur von 20° C bis 60° C, bei Bedarf Kühlen der Lösung auf eine Temperatur von minus 10° C bis plus 10° C und Abtrennen der dabei gebildeten Teilchen erfolgen.

Hierbei kann der Lösevorgang des als Ausgangsmaterial verwendetem Polylgucans bei Raumtemperatur oder höheren Temperaturen erfolgen.

Die Konzentration an linearem wasserunlöslichem Polyglucan inkl. ggf. verzweigtem Polysaccharid und weiteren Polymeren in dem Lösungsmittel kann je nach Bedarf in weiten Grenzen variieren. Vorzugsweise liegt sie in einem Bereich von 0,02 g /ml bis 1,0 g/ml, insbesondere von 0,05 g/ml bis 0,8 g/ml und besonders bevorzugt von 0,3 g/l bis 0,6 g/l.

Beispiele für Fällmittel sind Wasser, Dichlormethan, ein Gemisch aus Wasser und Dichlormethan, Gemische aus Wasser und Alkoholen wie Methanol, Ethanol, Isopropanol, wobei Wasser sowie ein Gemisch aus Wasser und Dichlomethan besonders bevorzugt sind.

Vorzugsweise wird das Verhältnis Lösungsmittel zu Fällmittel in einem Bereich von 1 : 1000 bis 1 : 4 (Teil Lösungsmittel / Teile Fällmittel), vorzugsweise 1 : 100 bis 1 : 10 und insbesondere 1 : 70 bis 1 : 30 ausgewählt.

Im allgemeinen ist es dabei unerheblich, in welcher Reihenfolge das Lösungsmittel und das Fällmittel zusammengebracht werden, z.B. ob das Fällmittel zum Lösungsmittel oder umgekehrt gegeben wird.

Wichtig ist jedoch, daß eine schnelle Durchmischung gewährleistet wird.

Der Fällprozeß kann relativ langsam bei tiefer Temperatur über Nacht durchgeführt werden.

Er kann durch Variation der Temperatur und des Fällmittels beeinflußt und gesteuert werden.

Falls gekühlt wird, muß sichergestellt sein, daß das Gemisch aus Lösungsmittel und Fällmittel liquide bleibt und nicht erstarrt.

Durch Mitverwendung geeigneter Zusatzstoffe läßt sich auf die Eigenschaften der Mikropartikel wie Größe, Oberflächenstruktur, Porosität etc. sowie auf die Prozeßführung Einfluß nehmen.

Geeignete Zusatzstoffe sind z. B. oberflächenaktive Stoffe wie Natriumdodecylsulfat, N-Methylgluconamid, Polysorbate (z. B. Tween (eingetragene Marke)), Alkylpolyglycolether, Ethylenoxid-Propylenoxid-Blockpolymere (z. B. Pluronic (eingetragene Marke)), Alkylpolyglycolethersulfate, generell Alkylsulfate und Fettsäureglycolester, und Zucker wie z. B. Fructose, Saccharose, Glucose, wasserlösliche Cellulose oder heißwasserlösliches Poly-alpha-D-Glucan wie z. B. native oder chemisch modifizierte Stärken, aus diesen Stärken gewonnene Poly-alpha-D-Glucane sowie stärkeanaloge Verbindungen.

Üblicherweise werden diese Zusatzstoffe dem Fällmittel zugesetzt. Die verwendete Menge hängt von dem jeweiligen Einzelfall sowie den erwünschten Partikeleigenschaften ab, wobei die Bestimmung der jeweils vorteilhaften Menge dem Fachmann geläufig ist.

Durch Zusatz von wasserlöslichen Cellulosederivaten zu dem Fällmittel lassen sich Mikropartikel mit besonders glatter Oberfläche erhalten, wobei die Tiefe der Unregelmäßigkeiten auf der Oberfläche der Mikropartikel im allgemeinen maximal 10 % des mittleren Durchmessers beträgt.

Beispiele von wasserlöslichen Cellulosederivaten sind Celluloseester und Celluloseether, deren Mischformen wie z.B. Hydroxypropylmethylcellulosen, Hydroxyethylcellulosen, Carboxymethylcellulosen, Celluloseacetate, Cellulosebutyrate, Cellulosepropionate, Celluloseacetobutyrate, Celluloseacetopropionate, Cellulosenitrate, Ethylcellulosen, Benzylcellulosen, Methylcellulosen etc.

Es können auch Mischungen von verschiedenen wasserlöslichen Cellulosederivaten eingesetzt werden.

Unter dem Begriff "wasserlösliche Cellulosederivate" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag GmbH, Frankfurt, 9. Auflage, 1987) unter die Kategorie sehr leicht löslich bis schwer löslich fallen.

Die Konzentration des wasserlöslichen Cellulosederivats in dem Fällmittel ist nicht weiter kritisch. Die Obergrenze ergibt sich zwangsläufig aus der resultierenden Vikosität und damit der Verarbeitbarkeit der entstehenden Lösung.

Als vorteilhaft haben sich Konzentrationen von 2 g (Cellulosederivat)/l (Fällmittel) bis 150 g/l, vorzugsweise von 5 g/l bis 80 g/l und insbesondere 8 g/l bis 20 g/l, erwiesen.

Der Anteil an besonders kleinen Partikeln mit einem mittleren Durchmesser von 1 nm bis 2 µm kann gesteigert werden, indem dem Fällmittel heißwasserlösliches Poly-alpha-D-glucan zugesetzt wird.

Es können hierfür dieselben Poly-alpha-D-glucanverbindungen eingesetzt werden wie sie auch im Zusammenhang mit dem linearen wasserunlöslichen Polyglucan genannt worden sind, soweit diese das Merkmal heißwasserlöslich erfüllen.

Bevorzugte Beispiele sind native oder chemisch modifizierte Stärken, aus diesen Stärken gewonnene Poly-alpha-D-glucane sowie stärkeanaloge Verbindungen.

Unter stärkeanalogen Verbindungen werden Verbindungen verstanden, die aus Poly-alpha-D-glucanen bestehen, aber nicht-pflanzlichen Ursprungs sind. Ein Beispiel hierfür ist Glykogen oder Dextran.

Die heißwasserlöslichen Poly-alpha-D-glucane können als Mischung aus einem linearen und einem verzweigten Anteil eingesetzt werden, wie sie z.B. in Stärke vorliegt. In diesem Fall sollte der Anteil an linearem Poly-alpha-D-glucan mehr als 15 Gew.%, vorzugsweise 50 bis 99,5 Gew.%, insbesondere 60 bis 90 Gew.% und ganz besonders bevorzugt 65 bis 80 Gew.%, bezogen auf die Gesamtmenge Poly-alpha-D-glucan im Fällmittel, betragen.

Sie können aber auch aus verzweigten Strukturen bestehen, wie sie z.B. im Amylopektin oder im Glykogen vorliegen.

Im Rahmen der vorliegenden Erfindung bedeutet "heißwasserlöslich", daß die Poly-alpha-D-glucane bei Raumtemperatur im wesentlich unlöslich sind, wobei vorzugsweise der gleiche Maßstab wie für den Begriff "wasserunlöslich" in Zusammenhang mit linearen Polysacchariden gilt.

Unter dem Begriff "Lösung" bzw. "Löslichkeit" werden insbesondere auch Suspensionen bzw. die Ausbildung von Suspensionen verstanden wie sie bei der Lösung von Stärke auftreten.

Beispielsweise zeigen die erfindungsgemäß bevorzugten heißwasserlöslichen Stärken bei Raumtemperatur so gut wie keine Löslichkeit in Wasser, während die sogenannten kaltwasserlöslichen Stärken unter diesen Bedingungen leichter löslich sind.

Die heißwasserlöslichen Stärken sind insbesondere dadurch charakterisiert, daß sie bei Erhitzen unter Eigendruck, z.B. in einem Autoklaven, auf eine Temperatur im Bereich von etwa 100 bis etwa 160 °C Lösungen bilden, wobei die jeweilige Temperatur von der Art der Stärke abhängt.

Beispielsweise kann Kartoffelstärke bei ca. 100 °C bis zur völligen Auflösung gekocht werden, während Maisstärke eine Temperatur von ca. 125 °C erfordert.

Für das erfindungsgemäße Verfahren werden die heißwasserlöslichen Poly-alpha-D-glucane dem Fällmittel vorzugsweise in maximaler Konzentration zugesetzt, d.h. es wird eine gesättigte Lösung hergestellt. Weitere geeignete Bereiche sind von mehr als 0,001 Gew.% bis 10 Gew.%, bevorzugt von 0,01 bis 2 Gew.% und insbesondere von 0,05 Gew.% bis 0,5 Gew.%, bezogen auf die eingesetzte Menge an Fällmittel.

Im Fall von thermoplastischen Polyglucan können die Zusatzstoffe vorteilhafter Weise als Weichmacher oder in Ergänzung zu den Weichmachern in die thermoplastische Mischung eingemischt werden, so daß eine trockene Pulvermischung vorliegt, die dann zu den Mikropartikeln verarbeitet werden kann, wobei der Bildungsprozeß der Mikropartikel auch in der endgültigen Rezeptur unter Einmischung der thermoplastischen Polyglucane erfolgen kann.

Eine ausführliche Beschreibung der hier verwendeten Mikropartikel, ihrer Herstellung und der dafür einsetzbaren wasserunlöslichen linearen Polyglucane findet sich in den prioritätsälteren, nicht vorveröffentlichten deutschen Patentanmeldungen der Anmelderin mit Aktenzeichen 197 37 481.6, 198 03 415.6, 198 16 070.4, 198 30 618.0, 198 27 978.7, 198 39 214.1, 198 39 216.8 und 198 39 212.5 auf die für die vorliegende Beschreibung bezug genommen wird. Die drei letzt genannten Anmeldungen betreffen insbesondere Verfahren zur Modifizierung der Partikelbeschaffenheit wie Oberflächenrauhikeit und Größe.

Weiter zeichnen sich die erfindungsgemäß eingesetzten Mikropartikel durch eine hohe Biokompatibilität aus.

Für die Biokompatibilität der erfindungsgemäß eingesetzten Mikropartikel ist insbesondere der naturidentische Charakter der für die Herstellung verwendeten wasserunlöslichen linearen Polyglucane sowie von deren Abbauprodukten von hoher Bedeutung.

Nachstehend wird die vorliegende Erfindung anhand einzelner Beispiele veranschaulicht.

## Beispiel 1

Herstellung von Mikropartikeln aus Poly(1,4-$\alpha$-D-glucan)

500 mg Poly(1,4-2-D-glucan) werden in 2,5 ml Dimethylsulfoxid (DMSO, p.a. von Riedel-de-Haen) bei ca. 70° C gelöst. Die DMSO-Lösung wird in 100 ml bidestilliertem Wasser unter Rühren eingetropft und die Lösung über Nacht bei 5° C aufbewahrt. Die feine milchige Suspension wird für 15 Minuten bei 3500 Umdrehungen pro Minute zentrifugiert und der Überstand abdekantiert. Der Bodensatz wird mit bidestilliertem Wasser aufgeschlämmt und erneut zentrifugiert. Der Vorgang wird noch zwei Mal wiederholt. Die Suspension wird im Anschluß gefriergetrocknet. Es werden 311 mg weißer Poly(1,4-$\alpha$-D-glucan) Partikel erhalten. Dies entspricht einer Ausbeute von 62 % farbloser Mikropartikel.

## Beispiel 2

Bewertung eines Sonnenschutzmittels enthaltend Mikropartikel nach Beispiel 1

Die Bewertung erfolgte nach den Vorschriften der COLIPA Sun Protection Factor Test - Methode.

Für die Untersuchung wurden Versuchspersonen ausgewählt, welche in der Verteilung ihrer UV-Empfindlichkeit der Anwendermehrzahl entsprachen, UVgewöhnte wurden ausgeschlossen.

Es wurden die Standardpräparate P1 und P3 (Fa. Beiersdorf AG) im Vergleich zu einer Zubereitung mit 5 Gew.% Mikropartikeln nach Beispiel 1 verwendet.

Die wesentlichen Untersuchungsparameter waren:

Verwendung eines Sonnensimulators nach Schrader (SU 2000, Hersteller PTI-Photon Technology GmbH), der mit einer 300 Watt Xenon-Kurzbogenlampe ein Licht mit einem repräsentativen Spektrum erzeugt, das auf die Intensitätswerte von 320 nm normiiert war. Der Lichtstrahl dieser Lampe wurde mit Hilfe von beweglichen Spiegeln auf sechs kreisförmig angeordnete Punkte gerichtet, so daß die homogene Bestrahlung mit 6 unterschiedlichen Lichtdosierungen innerhalb einer Sitzung möglich war.

Zur exakten Applikation der von der COLIPA-Norm geforderten Menge des Lichtschutzmittels von 2,0 +/- 0,04 mg/cm$^2$wurde das Lichtschutzmittel mit einer Plastikspritze auf einen Kunststoffspatel aufgetragen und mit diesem auf auf der zu bestrahlenden Fläche gleichmäßig verteilt.

Die Ablesung der Bestrahlungsfelder erfolgte nach 20 Stunden +/- 4 Stunden.

Der mittlere Lichtschutzfaktor (LSF) wurde nach folgender Formel errechnet, worin MED für minimale erythemale Dosis steht:

$$\text{LSF (COLIPA)} = \text{MED-Testfeld} / \text{MED-Leerfeld}$$

Es wurde ein mittlerer Lichtschutzfaktor von 6,15 mit einer Standardabweichung von 0,65 erhalten. Das Vertrauensintervall (V95%) lag innerhalb 20 % des Mittelwertes.

## Patentansprüche

1. Verwendung von sphärischen Mikropartikeln mit einem mittleren Durchmesser von 1 nm bis 100 μm, bestehend ganz oder teilweise aus mindestens einem wasserunlöslichen linearen Polyglucan mit einem Verzweigungsgrad in 6-Position von weniger als 0,5%, wobei die Partikel eine Dispersität in einem Bereich von 1,0 bis 10,0 aufweisen und separiert vorliegen, als wirksamer Bestandteil eines Sonnenschutzmittels

2. , Verwendung nach Anspruch 1, **dadurch gekennzeichnet, daß** die Tiefe von Unregelmäßigkeiten auf der Partikeloberfläche maximal 20 % des mittleren Durchmessers der sphärischen Mikropartikel beträgt.

3. Verwendung nach einem der vorhergehenden Ansprüche, **dadurch gekennzeichnet, daß** die Mikropartikel in dem Sonnenschutzmittel in einer Menge von 0,5 bis 70 Gew. % enthalten sind, bezogen auf das Gesamtgewicht des Sonnenschutzmittels.

4. Verwendung nach einem der vorhergehenden Ansprüche, **dadurch gekennzeichnet, daß** das mindestens eine wasserunlösliche Polyglucan Poly-1,4-α-D-glucan und/oder Poly-1,3-β-D-glucan, insbesondere Poly-1,4-α-D-glucan, ist.

5. Verwendung nach einem der vorhergehenden Ansprüche, **dadurch gekennzeichnet, daß** das mindestens eine wasserunlösliche lineare Polyglucan nach einer biotechnischen Methode erhalten worden ist.

6. Verwendung nach einem der vorhergehenden Ansprüche, **dadurch gekennzeichnet, daß** das mindestens eine wasserunlösliche Polyglucan biokatalytisch erzeugt worden ist.

7. Verwendung nach einem der vorhergehenden Ansprüche, **dadurch gekennzeichnet, daß** der Anteil an wasserunlöslichen linearen Polyglucan in den Mikropartikeln mindestens 70 % beträgt, bezogen auf den Gesamtgehalt an Polyglucan inkl. ggf. verzweigtem Polysaccharid und weiteren Polymeren.

8. Verwendung nach einem der vorhergehenden Ansprüche, **dadurch gekennzeichnet, daß** die Mikropartikel zu 100 % aus mindestens einem wasserunlöslichen linearen Polyglucan bestehen.

9. Verwendung nach einem der vorhergehenden Ansprüche, **dadurch gekennzeichnet, daß** das wasserunlösliche lineare Polyglucan Poly-1,4-α-D-glucan ist, das biotechnisch, insbesondere blokatalytisch, erzeugt worden ist.

## Claims

1. The use of spherical microparticles with an average diameter from 1 nm to 100 μm, which consist entirely or partially of at least one water-insoluble linear polyglucan, with a degree of branching of less than 0.5% in 6-position, wherein the particles have a dispersity in the range of 1,0 bei 10,0 and are separated from one another, as the active ingredient of a sun protection product.

2. The use of Claim 1, wherein the depth of irregularities an the particle surface is at most 20% of the average diameter of the spherical microparticles.

3. The use as claimed in any of the preceding claims, wherein the microparticles are present in the sun protection product in an amount of from 0.5 to 70% by weight, based an the total weight of the sun protection product.

4. The use as claimed in any of the preceding claims, wherein the at least one water-insoluble polyglucan is poly-1,4-α-D-glucan and or poly-1,3-β-D-glucan, in particular poly- 1,4-α-D-glucan.

5. The use as claimed in any of the preceding claims, wherein the at least one water-insoluble linear polyglucan has been obtained by a biotechnology method.

6. The use as claimed in any of the preceding claims, wherein the at least one water-insoluble polyglucan has been produced biocatalytically.

7. The use as claimed in any of the preceding claims, wherein the proportion of water-insoluble linear polyglucan in the microparticles is at least 70%, based an the total content of polyglucan including optionally branched polysaccharide and further polymers.

8. The use as claimed in any of the preceding claims, wherein the microparticles consist to an extent of 100% of at least one water-insoluble linear polyglucan.

9. The use as claimed in any of the preceding claims, wherein the water-insoluble linear polyglucan is poly-1,4-α-D-glucan which has been produced biotechnologically, in particular biocatalytically.

**Revendications**

1. Utilisation de microparticules sphériques ayant un diamètre moyen de 1 nm à 100 μm, consistant en totalité ou en partie en au moins un polyglucane linéaire insoluble dans l'eau ayant un degré de ramification en position 6 inférieur à 0,5 %, où les particules présentent une dispersité dans un domaine de 1,0 à 10,0 et sont présentes séparées, comme constituant actif d'un produit solaire.

2. Utilisation selon la revendication 1 **caractérisée en ce que** la profondeur des irrégularités sur la surface des particules représente au maximum 20 % du diamètre moyen des microparticules sphériques.

3. Utilisation selon l'une des revendications précédentes **caractérisée en ce que** les microparticules dans le produit solaire sont contenues en une quantité de 0,5 à 70 % en poids par rapport au poids total du produit solaire.

4. Utilisation selon l'une des revendications précédentes **caractérisée en ce que** le ou les polyglucanes insolubles dans l'eau est ou sont le poly-1,4-α-D-glucane et/ou le poly-1,3-β-D-glucane, en particulier le poly-1,4-α-D-glucane.

5. Utilisation selon l'une des revendications précédentes **caractérisée en ce que** le ou les polyglucanes linéaires insolubles dans l'eau ont été obtenus selon une méthode biotechnique.

6. Utilisation selon l'une des revendications précédentes **caractérisée en ce que** le ou les polyglucanes insolubles dans l'eau ont été produits biocatalytiquement.

7. Utilisation selon l'une des revendications précédentes **caractérisée en ce que** la proportion de polyglucane linéaire insoluble dans l'eau dans les microparticules est d'au moins 70 %, par rapport à la teneur totale en polyglucane y compris un polysaccharide éventuellement ramifié et d'autres polymères.

8. Utilisation selon l'une des revendications précédentes **caractérisée en ce que** les microparticules consistent à 100 % en au moins un polyglucane linéaire insoluble dans l'eau.

9. Utilisation selon l'une des revendications précédentes **caractérisée en ce que** le polyglucane linéaire insoluble dans l'eau est le poly-1,4-α-D-glucane qui a été produit biotechniquement, en particulier biocatalytiquement.